(19) Europäisches Patentamt
European Patent Office
Office européen des brevets

(11) Numéro de publication: **0 193 450**
**A2**

(12) # DEMANDE DE BREVET EUROPEEN

(21) Numéro de dépôt: **86400300.9**

(51) Int. Cl.⁴: **C 07 C 119/14**

(22) Date de dépôt: **12.02.86**

(30) Priorité: **26.02.85 FR 8502724**

(43) Date de publication de la demande:
**03.09.86 Bulletin 86/36**

(84) Etats contractants désignés:
**AT BE CH DE FR GB IT LI LU NL SE**

(71) Demandeur: **SYNTHELABO**
**58, rue de la Glacière**
**F-75621 Paris Cedex 13(FR)**

(72) Inventeur: **Raizon, Bernard**
**49, rue Gabriel Péri**
**F-91270 Vigneux(FR)**

(72) Inventeur: **Rossey, Guy**
**8, Square Lebrun Voisins-le-Bretonneux**
**F-78180 Montigny-Le-Bretonneux(FR)**

(72) Inventeur: **Wick, Alexander**
**10, Bd des Plants**
**F-78800 St Nom-de-Breteche(FR)**

(74) Mandataire: **Thouret-Lemaitre, Elisabeth et al,**
**Service Brevets - SYNTHELABO 58, rue de la Glacière**
**F-75621 Paris Cedex 13(FR)**

(54) **Procédé de préparation de diphénylazométhines.**

(57) Préparation de diphénylazométhines de formule (II)

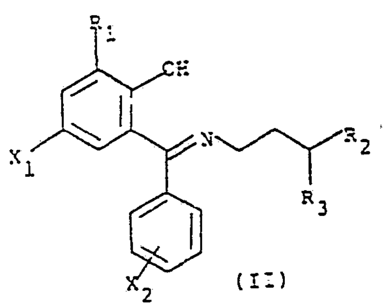

(II)

dans laquelle $X_1$ et $X_2$ représentent chacun, indépendamment l'un de l'autre, un atome d'hydrogène ou un atome d'halogéne ou un radical méthyle, $R_1$ est H ou un radical $(C_{1-4})$alkyle droit ou ramifié, $R_2$ est le radical COOH, un radical $(C_{1-4})$alkyle droit ou. ramifié, le radical $CONH_2$ ou un radical $COO(C_{1-4})$ alkyle droit ou ramifié, et $R_3$ représente un atome d'hydrogène ou le radical méthyle, par décarboxylation des composés correspondants portant un radical COOH sur le carbone voisin de l'atome d'azote.

EP 0 193 450 A2

Croydon Printing Company Ltd.

La présente invention a pour objet la préparation de diphénylazométhines utiles en thérapeutique et déjà décrites par la Demanderesse dans ses brevets français 75.24065, 76.21922, 80.03009, 81.04478, 81.21559, 82.18193, 82.18194, 82.19981 et 83.07863.

Le procédé de l'invention consiste à décarboxyler par chauffage au reflux dans un solvant, tel que le toluène, pendant un temps variable, les composés répondant à la formule (I) donnée en annexe 1,
dans laquelle
$X_1$ et $X_2$ représentent chacun, indépendamment l'un de l'autre, un atome d'hydrogène ou un atome d'halogène ou un radical méthyle,
$R_1$ est H ou un radical $(C_{1-4})$alkyle droit ou ramifié,
$R_2$ est le radical COOH, un radical $(C_{1-4})$alkyle droit ou ramifié, le radical $CONH_2$ ou un radical $COO(C_{1-4})$alkyle droit ou ramifié, et
$R_3$ est un atome d'hydrogène ou le radical méthyle.

Les diphénylazométhines (II) ainsi obtenues sont utiles en thérapeutique, comme antiépileptiques, antidépresseurs, antidiskynétiques,...

Les composés (I) peuvent être préparés selon le schéma réactionnel donné en annexe 1.
On fait réagir une benzophénone (III) avec un acide aminé (IV) dans un solvant, en présence d'une base ; à une température de 60 à 80°C.

Les composés (I) nouveaux, utiles comme intermédiaires de synthèse, répondent à la formule (I) donnée en annexe 1,
dans laquelle
$X_1$ et $X_2$ représentent chacun, indépendamment l'un de l'autre, un atome d'hydrogène ou un atome d'halogène ou un radical méthyle,
$R_1$ est H ou un radical $(C_{1-4})$alkyle droit ou ramifié,
$R_2$ est le radical COOH, un radical $(C_{1-4})$alkyle droit ou ramifié, le radical $CONH_2$ ou un radical $COO(C_{1-4})$alkyle

droit ou ramifié, et

$R_3$ est un atome d'hydrogène ou le radical méthyle,

à l'exception des composés pour lesquels

. $X_1$ = H, $CH_3$ ou Cl, $X_2$ = H, $R_1$ = H, $R_2$ = $CONH_2$ et $R_3$ = H

et

. $X_1$ = H, $CH_3$ ou Cl, $X_2$ = H, $R_1$ = H, $R_2$ = $R_3$ = $CH_3$.

Le procédé de l'invention permet d'obtenir les composés (II) avec un excellent rendement à partir des composés (I).

Les exemples suivants illustrent l'invention.
Les analyses et les spectres IR et RMN confirment la structure des composés.

Exemple 1 (Préparation d'un composé 1)
Acide amino-5 [((chloro-5 hydroxy-2 méthyl-3 phényl)(chloro-4 phényl)méthylène)amino]-2 oxo-5 pentanoïque.

On évapore à sec sous léger vide (bain à 80°C) un mélange de 10 g (0,0355 mole), de (chloro-5 hydroxy-2 méthyl-3 phényl)(chloro-4 phényl)méthanone, de 5,2 g (0,0355 mole) de glutamine et de 3 g (0,0355 mole) de bicarbonate de sodium dans 300 ml de méthanol. Puis on agite le résidu à 80°C, sous pression réduite pendant 15 minutes. On ajoute alors 300 ml de méthanol et évapore dans les mêmes conditions que précédemment. On recommence 5 fois cette opération. On dissout le résidu final refroidi dans 1,2 l d'eau et on acidifie à pH4, en agitant, par introduction d'acide citrique. On extrait au chloroforme, lave la phase organique à l'eau, décante, sèche sur $MgSO_4$, filtre et évapore à sec. Le solide obtenu est entraîné sur fritté avec 100 ml de pentane. On filtre, essore et recristallise 2 fois dans de l'acétate d'éthyle avec traitement au charbon végétal. On filtre les cristaux, essore, lave avec le minimum d'éther, essore et sèche au dessiccateur chauffant à 60°C. Le composé obtenu fond à 177-178°C.

Exemple 2 (Préparation d'un composé II)

[[(chloro-5 hydroxy-2 méthyl-3 phényl)(chloro-4 phényl)-méthylène] amino]-4 butanamide.

On chauffe à la température de reflux, pendant 15 minutes, une solution de 60 mg du composé obtenu dans l'exemple 1 dans 7,5 ml de toluène. On évapore le mélange réactionnel à sec et fait cristalliser le résidu dans 10 ml d'éther. On filtre les cristaux, essore et sèche au dessiccateur chauffant à 60°C en présence de $P_2O_5$.

Le produit obtenu fond à 154-155°C.

Dans le tableau suivant sont représentés les composés de l'invention préparés à titre d'exemples.

Tableau

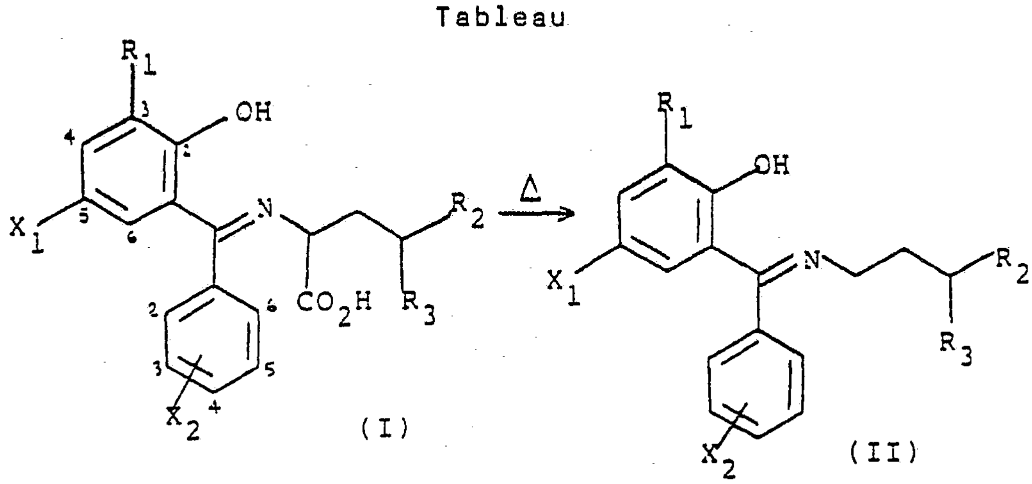

(I)

(II)

| Composé | $X_1$ | $X_2$ | $R_1$ | $R_2$ | $R_3$ | F(°C) composé(I) | F(°C) composé(II) |
|---|---|---|---|---|---|---|---|
| 1 | Cl-5 | Cl-4 | $CH_3$ | $CONH_2$ | H | 177-178 | 154-155 |
| 2 | Cl-5 | Cl-4 | $CH_3$ | COOH | H | 139-140 | 131-132 |
| 3 | Cl-5 | Cl-4 | $nC_3H_7$ | $CONH_2$ | H | 152-153 | 129-130 |
| 4 | F-5 | Cl-4 | H | $CONH_2$ | H | 126-127 | 143-144 |
| 5 | Cl-5 | Cl-2 | H | $CH_3$ | H | 165-166 | 54-55 |
| 6 | Cl-5 | Cl-2 | H | $iC_3H_7$ | $CH_3$ | 144-145 | 47-48 |

Annexe 1

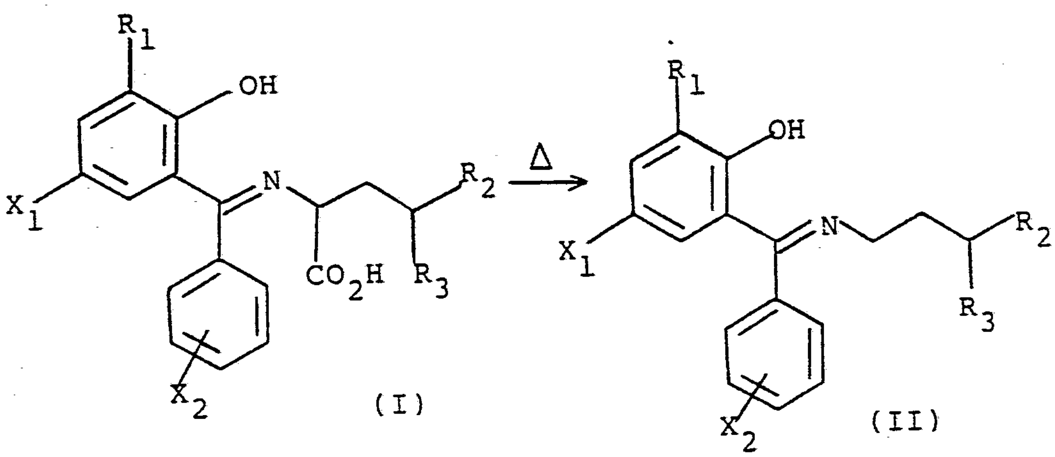


(I) (II)

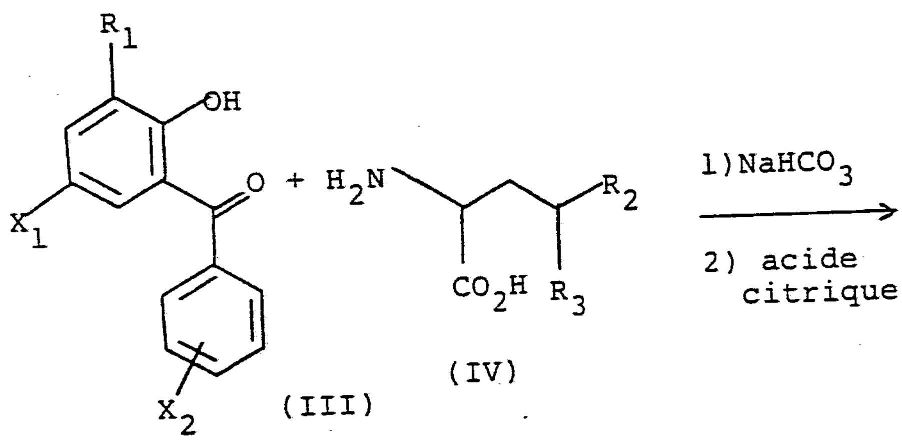


(III) (IV) (I)

Revendications pour les états contractants :
BE, CH, DE, FR, GB, IT, LI, NL, SE.

1. Procédé de préparation des diphénylazométhines répondant à la formule (II)

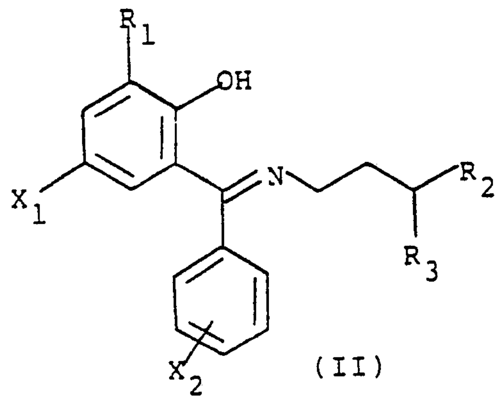

(II)

dans laquelle
$X_1$ et $X_2$ représentent chacun, indépendamment l'un de l'autre, un atome d'hydrogène ou un atome d'halogène ou un radical méthyle,
$R_1$ est H ou un radical $(C_{1-4})$alkyle droit ou ramifié,
$R_2$ est le radical COOH, un radical $(C_{1-4})$alkyle droit ou ramifié, le radical $CONH_2$ ou un radical $COO(C_{1-4})$alkyle droit ou ramifié, et
$R_3$ représente un atome d'hydrogène ou le radical méthyle,
par décarboxylation des composés de formule (I)

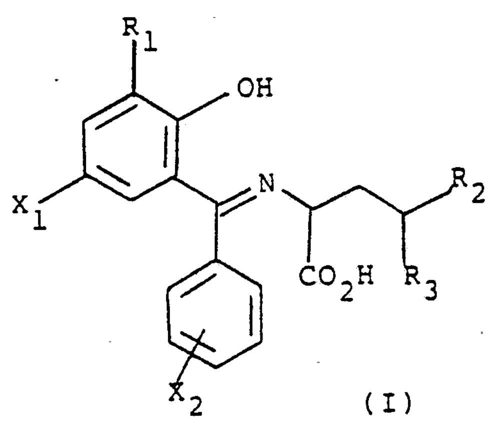

(I)

dans laquelle les radicaux $X_1$, $X_2$, $R_1$, $R_2$ et $R_3$ ont les significations données ci-dessus.

2. Composés, utiles pour la préparation des composés de formule (II), répondant à la formule (I)

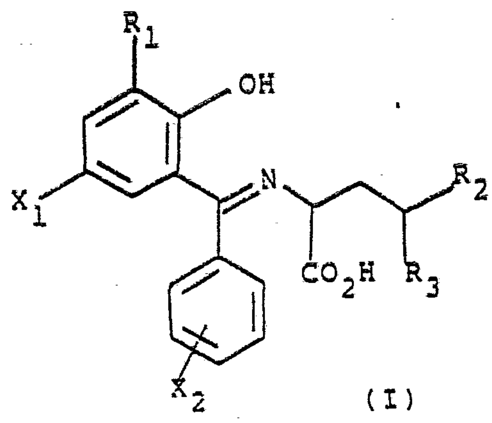

(I)

dans laquelle

$X_1$ et $X_2$ représentent chacun, indépendamment l'un de l'autre, un atome d'hydrogène ou un atome d'halogène ou un radical méthyle,

$R_1$ est H ou un radical $(C_{1-4})$alkyle droit ou ramifié,

$R_2$ est le radical COOH, un radical $(C_{1-4})$alkyle droit ou ramifié, le radical $CONH_2$ ou un radical $COO(C_{1-4})$alkyle droit ou ramifié, et

$R_3$ représente un atome d'hydrogène ou le radical méthyle,

à l'exception des composés pour lesquels

. $X_1$ = H, $CH_3$ ou Cl, $X_2$ = H, $R_1$ = H, $R_2$ = $CONH_2$ et $R_3$ = H et

. $X_1$ = H, $CH_3$ ou Cl, $X_2$ = H, $R_1$ = H, $R_2$ = $R_3$ = $CH_3$.

Revendication pour l'état contractant : AT.

Procédé de préparation des diphénylazométhines répondant à la formule (II)

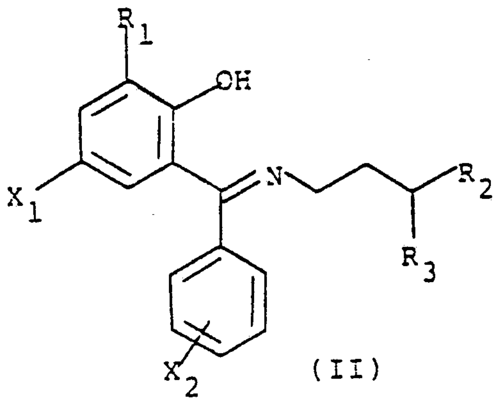

(II)

dans laquelle

$X_1$ et $X_2$ représentent chacun, indépendamment l'un de l'autre, un atome d'hydrogène ou un atome d'halogène ou un radical méthyle,

$R_1$ est H ou un radical $(C_{1-4})$alkyle droit ou ramifié,

$R_2$ est le radical COOH, un radical $(C_{1-4})$alkyle droit ou ramifié, le radical $CONH_2$ ou un radical $COO(C_{1-4})$alkyle droit ou ramifié, et

$R_3$ représente un atome d'hydrogène ou le radical méthyle,

par décarboxylation des composés de formule (I)

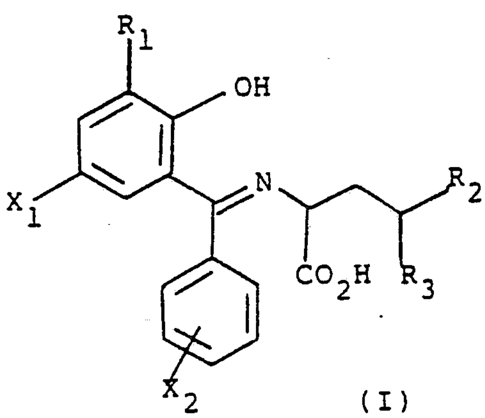

(I)

dans laquelle les radicaux $X_1$, $X_2$, $R_1$, $R_2$ et $R_3$ ont les significations données ci-dessus.